# EUROPÄISCHE PATENTSCHRIFT

(11) **EP 1 230 230 B1**
(45) Veröffentlichungstag und Bekanntmachung des Hinweises auf die Patenterteilung: **17.09.2003**
(21) Anmeldenummer: 00974437.6
(22) Anmeldetag: 21.10.2000
(51) Int. Cl.: C07D 277/60, A61K 31/425, A61P 3/04, A61P 3/10

(54) **INDENO-DIHYDROTHIAZOL DERIVATE, DEREN HERSTELLUNG UND DEREN VERWENDUNG ALS ANOREKTISCHE ARZNEIMITTEL**
INDENO-DIHYDROTHIAZOLE DERIVATIVES, THE PRODUCTION THEREOF AND THEIR USE AS ANORECTIC MEDICAMENTS
DERIVES D'INDENO-DIHYDROTHIAZOL, LEUR PREPARATION ET LEUR UTILISATION COMME MEDICAMENTS ANOREXIGENES

(30) Priorität: 05.11.1999 DE 19953205; 17.03.2000 DE 10013306
(43) Veröffentlichungstag der Anmeldung: 14.08.2002
(73) Patentinhaber: Aventis Pharma Deutschland GmbH, 65929 Frankfurt am Main (DE)
(72) Erfinder: JAEHNE, Gerhard, 65929 Frankfurt (DE); GOSSEL, Matthias, 65719 Hofheim (DE); LANG, Hans-Jochen, 65719 Hofheim (DE); BICKEL, Martin, 61348 Bad Homburg (DE)
(86) Internationale Anmeldenummer: EP0010396
(87) Internationale Veröffentlichungsnummer: WO01032638

(56) Entgegenhaltungen:
- WO-A-00/04006
- WO-A-00/18749
- WO-A-00/51994
- DE-A- 2 640 358
- US-A- 3 507 868

## Beschreibung

Die Erfindung betrifft polycyclische Dihydrothiazole sowie deren physiologisch verträgliche Salze und physiologisch funktionelle Derivate.

Es sind bereits Thiazolidinderivate mit anorektischer Wirkung im Stand der Technik beschrieben (Österreichisches Patent Nr. 365181).

US 3,507,868 offenbart Indeno-, Naphto- und Benzocyclohepta[1,2-d]dihydrothiazole, die im Gegensatz zu den erfindungsgemäßen Verbindungen einen zusätzlichen ankondensierten Ring am Thiazolring besitzen.

DE 2640358 offenbart Indeno- und Naphtocyclohepta-[1,2-d]dihydrothiazole die sich durch den Substituenten R3 am Stickstoff und den Substituenten -NR4 von den den erfindungsgemäßen Verbindungen unterscheiden.

WO 00 18749 offenbart Indeno- und Naphto- und Benzocycloheptadihydrothiazole die sich durch den Substituenten R4 von den erfindungsgemäßen Verbindungen unterscheiden.

Der Erfindung lag die Aufgabe zugrunde, weitere Verbindungen zur Verfügung zu stellen, die eine therapeutisch verwertbare anorektische Wirkung entfalten.

Die Erfindung betrifft daher Verbindungen der Formel I, 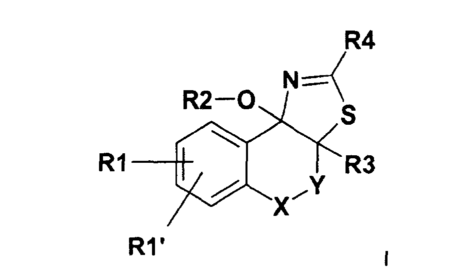 worin bedeuten
- Y: eine direkte Bindung;
- X: CH₂;
- R1, R1': unabhängig voneinander H, F, Cl, Br, J, (C₁-C₆)-Alkyl;
- R2: H, (C₁-C₆)-Alkyl;
- R3: H, F;
- R4: (C₈-C₁₆)-Cycloalkyl oder (CH₂)ₙ-A-R8, wobei n = 1 - 6 sein kann, wobei die Gruppe -(CH₂)₁₋₆-O-CH₂-Phenyl, worin Phenyl unsubstituiert ist, ausgenommen ist;
- A: O, S;
- R8: (C₅-C₂₄)-Alkyl, oder (CH₂)ₘ-Aryl, wobei m = 0-6 und Aryl gleich Phenyl ist
und der Arylteil bis zu zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, SO₂-NH₂, SO₂-NH(C₁-C₈-Alkyl), SO₂-N(C₁-C₈-Alkyl)₂, SO₂-NH(C₃-C₈-Cycloalkyl), SO₂-N(C₃-C₈-Cycloalkyl)₂, (CH₂)m-SO₂-NH₂, (CH₂)m-SO₂-NH-(C₁-C₆)-Alkyl, (CH₂)m-SO₂-N((C₁-C₆)-Alkyl)₂, wobei m = 1-6 sein kann, SO₂-N(=CH-N(CH₃)₂), (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH(C₁-C₆)-Alkyl, N(C₁-C₆-Alkyl)₂, NH-CO-(C₁-C₆)-Alky, NH-CO-Phenyl, NH-SO₂-(C₁-C₈-Alkyl), N(C₁-C₆-Alkyl)-SO₂-(C₁-C₈-Alkyl), NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl oder CONH₂ substituiert sein kann, Pyrrolidin-1-yl, Morpholin-1-yl, Piperidin-1-yl, Piperazin-1-yl, 4-Methylpiperazin-1-yl, (CH₂)p-Phenyl, O-(CH₂)ₚ-Phenyl, S-(CH₂)ₚ-Phenyl oder SO₂-(CH₂)ₚ-Phenyl, wobei p = 0-3 sein kann;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R1', R2, R3, R4, R8 und A können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-,
Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natriumund Kaliumsalze) und Erdalkalisalze (wie Magnesium- und Caiciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

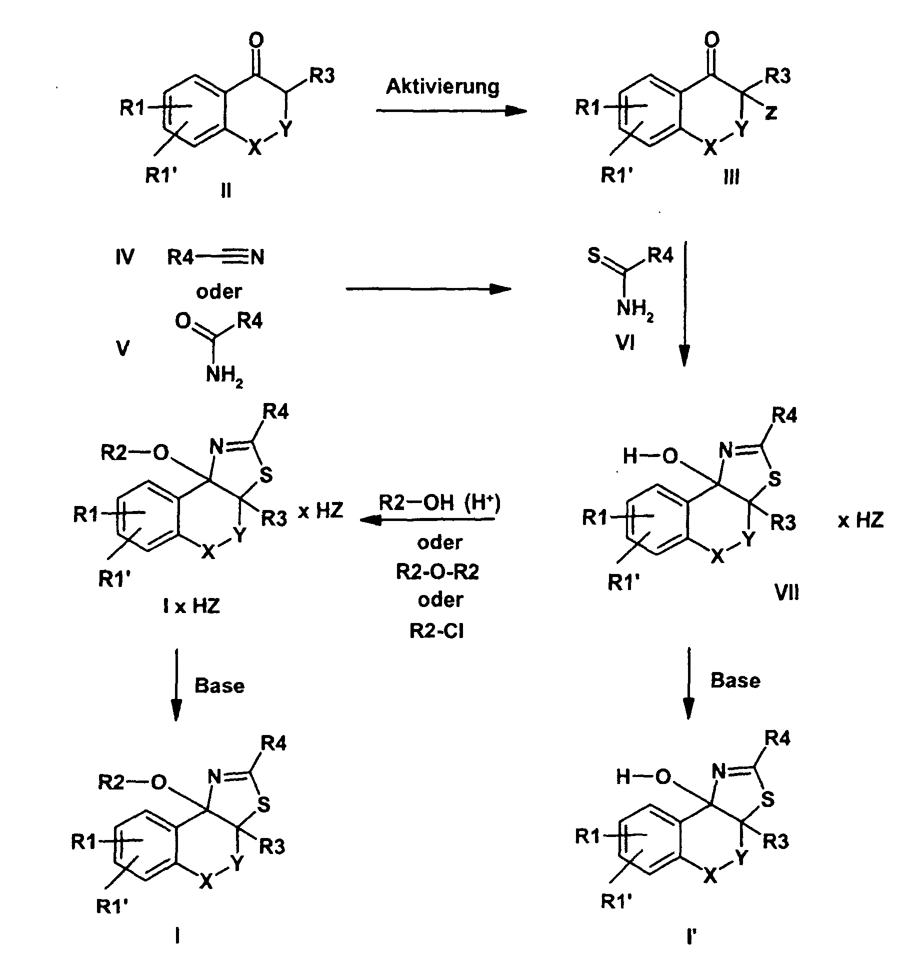

Dazu werden Verbindungen der allgemeinen Formel II, 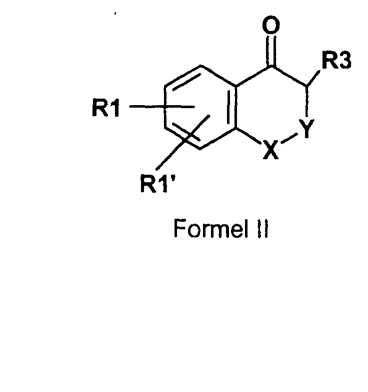 worin R1, R1', R3 und X und Y die angegebene Bedeutung besitzen, aktiviert und in eine Verbindung der Formel III überführt, worin Z für den Rest eines aktivierten Esters einer anorganischen oder organischen Säure steht.

Die Verbindungen der Formel III werden weiter mit Thioamiden der Formel VI 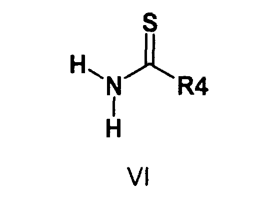 worin R4 die angegebene Bedeutung besitzt, zu Verbindungen der Formel VII bzw. I' umgesetzt, wobei gegebenenfalls die Verbindungen der allgemeinen Formel I' mit organischen oder anorganischen Säuren in ihre Säureadditionssalze der Formel VII oder erhaltene Salze der Formel VII mit organischen oder anorganischen Basen in die freien basischen Verbindungen der Formel I' überführt werden.

Als anorganische Säuren kommen beispielsweise in Betracht: Halogenwasserstoffsäuren wie Chlorwasserstoffsäure und Bromwasserstoffsäure, sowie Schwefelsäure, Phosphorsäure und Amidosulfonsäure.

Als organische Säuren seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen III mit den Thioamiden VI im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen - 10°C und 150°C, bevorzugt zwischen 30°C und 100°C, durchgeführt werden. Als besonders günstig erwies sich in der Regel ein Temperaturbereich zwischen 50°C und 90°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen VII in Form ihrer Säureadditionssalze im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen VII, ggf. nach Konzentrierung der Reaktionslösung, mit einem der genannten Fällungsmittel ausfällen. Femer kann man die Lösung des Reaktionsgemisches auch vorteilhaft in die Lösung eines der genannten Fällungsmittel unter Rühren einfiltrieren. Da die Reaktion der Verbindungen III mit den Thioamiden VI praktisch quantitativ abläuft, sind die erhaltenen Rohprodukte meistens bereits analytisch rein. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I' ein Säureadditionsprodukt der Formel VII so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisiopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I' können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.

Die Reaktion der Verbindungen der Formel II mit den Thioamiden der Formel VI kann auch so durchgeführt werden, daß man zur Reaktionsmischung eine wenigstens äquimolare Menge einer Base, wie z.B. Triethylamin, zufügt und die so erhaltenen Verbindungen I' anschließend gegebenenfalls in ihre Säureadditionsprodukte VII überführt.

In den Verbindungen der Formel III kommen als Rest eines aktivierten Esters Z beispielsweise in Frage: Cl, Br, J, O-C(O)-(C₆H₄)-4-NO₂, O-SO₂-CH₃, O-SO₂-CF₃, O-SO₂-(C₆H₄)-4-CH₃, O-SO₂-C₆H₄.

Die Säureadditionsprodukte VII und I x HZ können durch Behandlung mit Basen zu den Verbindungen der allgemeinen Formel I und I' umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Thioamide der allgemeinen Formel VI sind entweder kommerziell erhältlich oder können z.B. durch Umsetzung des entsprechenden Carbonsäureamids V mit Phosphorpentasulfid in Pyridin (R. N. Hurd, G. Delameter, Chem. Rev. 61, 45 (1961)), oder mit Lawesson's Reagenz in Toluol, Pyridin, Hexamethylphosphorsäurtriamid [Scheibye, Pedersen und Lawesson: Bull. Soc. Chim. Belges 87, 229 (1978)], vorzugsweise in einem Gemisch von Tetrahydrofuran mit 1,3-Dimethyl-3,4,5,6-tetrahydro-2(1H)-pyrimidinon oder 1,3-Dimethyl-2-Imidazolidinon gewonnen werden. Hydroxy-, Amino- oder zusätzliche Carbonylfunktionen werden dabei zweckmäßigerweise mit einer wiederabspaltbaren Schutzfunktion, wie z. B. einem Benzyl-, tert.Butyloxycarbonyl-, Benzyloxycarbonylrest bzw. durch Überführung in ein, gegebenfalls cyclisches, Acetal überführt. Methoden dazu sind z. B. beschrieben in Th. W. Greene und P. G. M. Wuts, Protective Groups in Organic Synthesis, Second Edition, 1991, John Wiley & Sons, New York.

Thioamide der Formel VI sind auch erhältlich, indem man Nitrile der allgemeinen Formel IV

**N**≡-**R4** Formel IV

mit Schwefelwasserstoff (Houben-Weyl IX, 762) oder Thioacetamid (E. C. Taylor, J. A. Zoltewicz, J. Am. Chem. Soc. 82, 2656 (1960)) oder O,O-Diethyldithiophosphorsäure umsetzt. Die Umsetzungen mit Schwefelwasserstoff werden vorzugsweise in einem organischen Lösemittel wie Methanol oder Ethanol, die mit Thioacetamid in einem Lösemittel wie Dimethylformamid unter Hinzufügung von Salzsäure, die mit O,O-Diethyl-dithiophosphorsäure in einem Lösemittel wie Essigsäureethylester unter sauren, z.B. HCI, Bedingungen bei Raumtemperatur oder unter Erwärmung durchgeführt.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

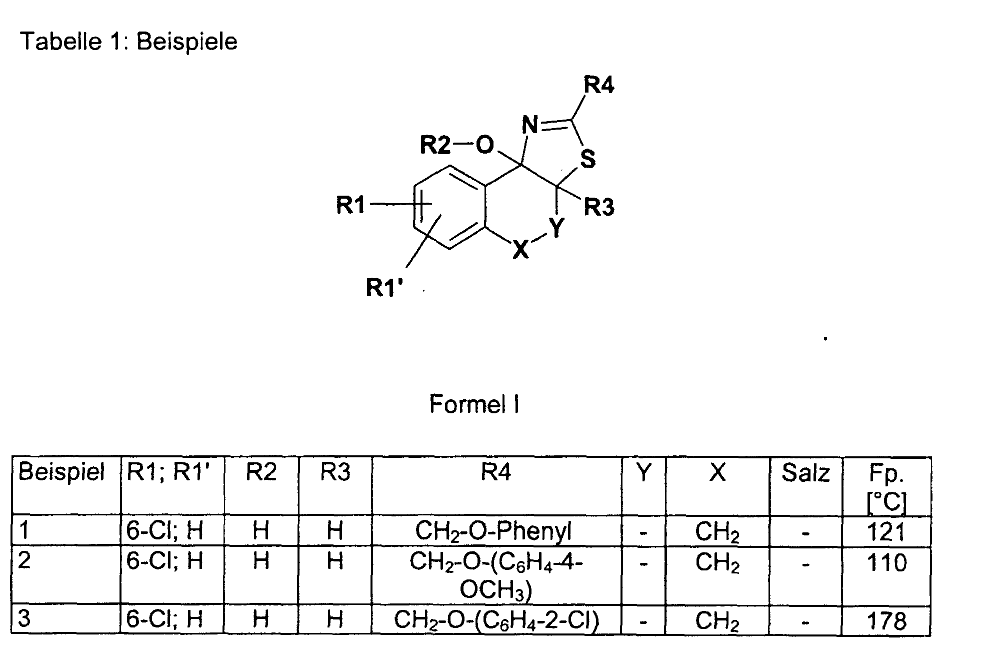

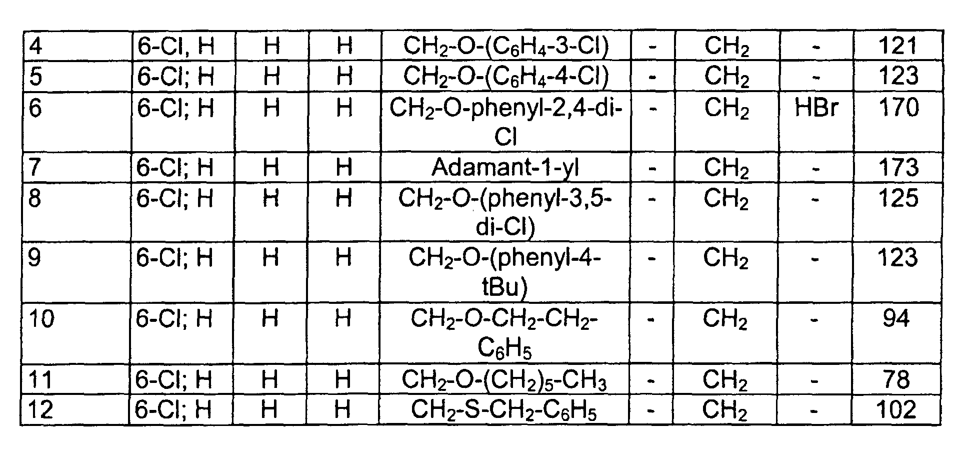

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

Die Wirksamkeit der Verbindungen wurde wie folgt getestet:

### Biologisches Prüfmodell:

Die Prüfung der anorektischen Wirkung erfolgte an männlichen oder weiblichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

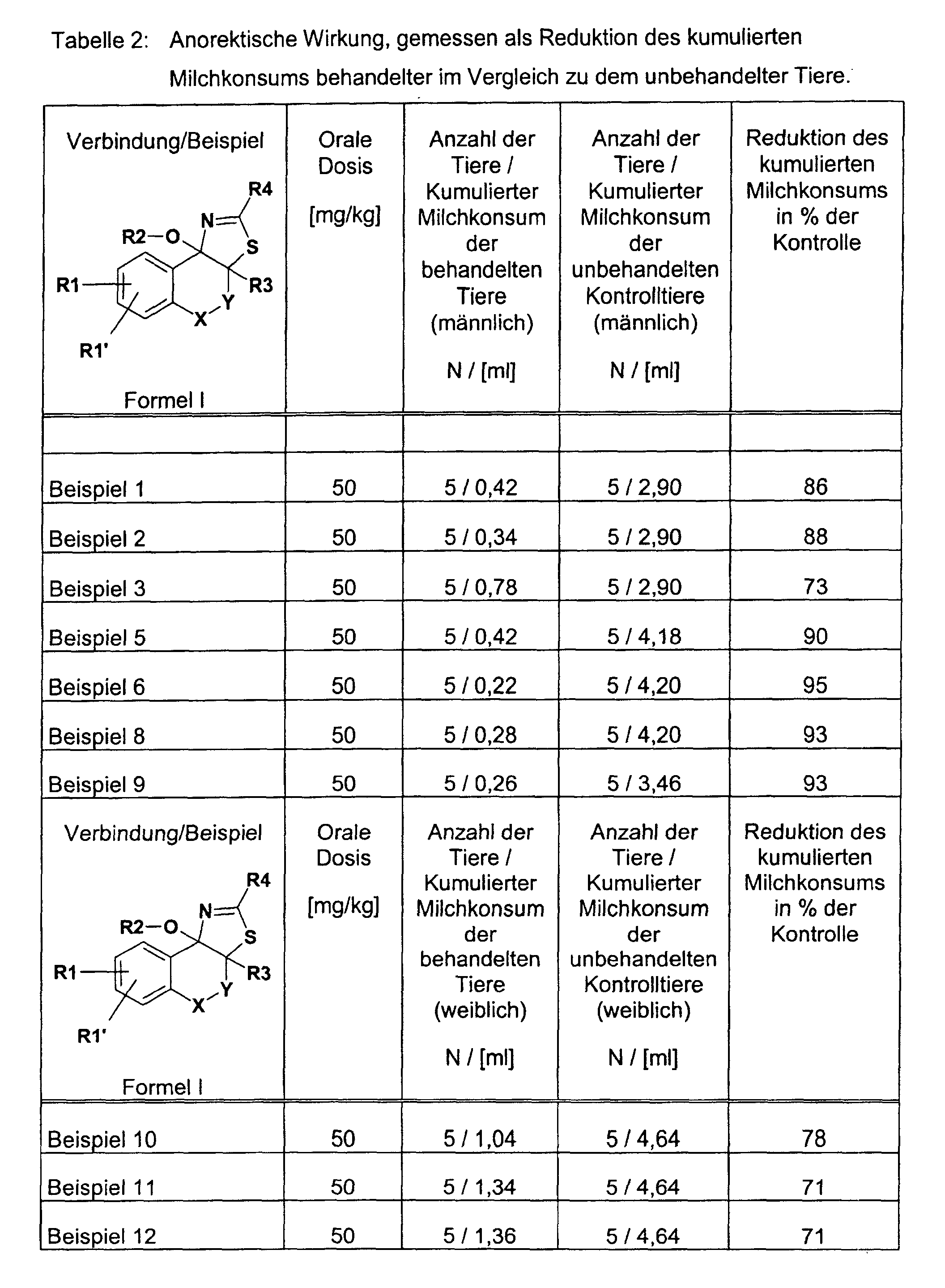

Aus der Tabelle ist abzulesen, daß die Verbindungen der Formel 1 eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1 (Verbindung 1):

### 6-Chlor-2-phenoxymethyl-8,8a-dihydro-indeno[1,2-d]thiazol-3a-ol:

a) 2-Brom-5-chlor-indan-1-on:
   10 g (0.06 Mol) 5-Chlor-indan-1-on werden bei Raumtemperatur in 120 ml Eisessig unter Rühren gelöst. Man tropft 0.05 ml einer 48%igen Lösung von HBr in Wasser und anschließend 3.074 ml (0.06 mol) Brom, gelöst in 25 ml Eisessig, zu. Nach 2 h Rühren bei Raumtemperatur ist die Reaktion beendet (DC-Kontrolle).Die Lösung des Rohproduktes wird unter Rühren langsam in 300 ml Eiswasser eingetropft. Das ausgefallene Rohprodukt wird abgesaugt und gründlich mit Wasser gewaschen. Der feuchte Rückstand wird mit Essigsäureethylester vom Filter abgelöst und die Phasen des Filtrats getrennt. Die organische Phase wird über Natriumsulfat getrocknet, filtriert und im Vakuum eingeengt. Der Rückstand wird in 120 ml n-Heptan heiß gelöst; die heiße Lösung wird über ein Faltenfilter filtriert und die Lösung dann bei 0°C der Kristallisation überlassen. Das kristallisierte Produkt wird abgesaugt und im Vakuum getrocknet.
   Fp.: 94-96°C
b) 6-Chlor-2-phenoxymethyl-8,8a-dihydro-indeno[1,2-d]thiazol-3a-ol:
   0.49 g 2-Brom-5-chlor-indan-1-on werden bei Raumtemperatur in 10 ml trockenem Aceton gelöst und mit 335 mg 2-Phenoxy-thioacetamid versetzt. Man rührt 6 h bei Raumtemperatur, saugt das kristallisierte Hydrobromid des Produktes ab, wäscht den Rückstand mit Aceton und trocknet ihn im Vakuum. Die freie Base wird erhalten, indem das Salz in eine Mischung aus 30 ml Essigsäureethylester und 20 ml gesättigter Natriumhydrogencarbonatlösung eingetragen und 20 min. gerührt wird. Die organische Phase wird abgetrennt, mit gesättigter Kochsalzlösung gewaschen und über Magnesiumsulfat getrocknet. Man filtriert und engt das Filtrat im Vakuum ein. Es wird 6-Chlor-2-phenoxymethyl-8,8a-dihydro-indeno[1,2-d]thiazol-3a-ol mit dem Schmelzpunkt 121°C erhalten.

### Beispiel 2 (Verbindung 6):

### 6-Chlor-2-(2,4-dichlor-phenoxymethyl)-8,8a-dihydro-indeno[1,2-d]thiazol-3a- ol Hydrobromid:

0.25 g 2-Brom-5-chlor-indan-1-on und 0.24 g 2-(2,4-Dichlor-phenoxy)-thioacetamid werden bei Raumtemperatur in 5 ml trockenem Aceton gelöst und 12 h bei Raumtemperatur gerührt. Der ausgefallene Niederschlag wird abgesaugt, mit Aceton gewaschen und im Hochvakuum getrocknet. Man erhält das Hydrobromid des 6-Chlor-2-(2,4-dichlor-phenoxymethyl)-8,8a-dihydro-indeno[1,2-d]thiazol-3aols mit dem Schmelzpunkt 170°C (Zersetzung).

### Beispiel 3 (Verbindung 9):

### 2-(4-tert-Butyl-phenoxymethyl)-6-chlor-8,8a-dihydro-indeno[1,2-d]thiazol-3a-ol:

0.49 g 2-Brom-5-chlor-indan-1-on und 0.45 g 2-(4-tert-Butyl-phenoxy)-thioacetamid werden bei Raumtemperatur in 10 ml trockenem Aceton gelöst und 12 h bei Raumtemperatur gerührt. Das ausgefallene Hydrobromid wird abgesaugt und mit Aceton gewaschen. Der Rückstand wird in wenig Essigsäureethylester suspendiert und mit gesättigter Natriumhydrogencarbonatlösung behandelt. Die organische Phase wird abgetrennt, über Magnesiumsulfat getrocknet, filtriert und im Vakuum eingeengt. Man erhält 2-(4-tert-Butyl-phenoxymethyl)-6-chlor-8,8adihydro-indeno[1,2-d]thiazol-3a-ol mit dem Schmelzpunkt 122-124°C.

### Beispiel 4 (Verbindung 12):

### 2-Benzylsulfanylmethyl-6-chlor-8,8a-dihydro-indeno[1,2-d]thiazol-3a-ol:

### a) 2-Benzylsulfanyl-thioacetamid:

1.2 g Benzylsulfanyl-acetonitril werden bei Raumtemperatur in 10 ml trockenem Ethanol gelöst, mit 1.25 ml Diethyldithiophosphat versetzt und 6 h unter Rückfluß gerührt. Die abgekühlte Reaktionslösung wird im Vakuum eingeengt und über Kieselgel mit Essigsäureethylester/n-Heptan 1/2 chromatographisch gereinigt. Das so gewonnene 2-Benzylsulfanyl-thioacetamid wird in die nächste Stufe eingesetzt.

### b) 2-Benzylsulfanylmethyl-6-chlor-8,8a-dihydro-indeno[1,2-d]thiazol-3a-ol:

0.54 g 2-Benzylsulfanyl-thioacetamid werden bei Raumtemperatur in 10 ml trockenem Aceton gelöst, mit 0.67 g 2-Brom-5-chlor-indan-1-on versetzt und 4 h bei Raumtemperatur gerührt. Der gebildete Niederschlag wird abgesaugt, mit Aceton gewaschen, in Essigsäureethylester aufgenommen, mit ges. Natriumhydrogencarbonatlösung versetzt und zweimal ausgeschüttelt. Die organische Phase wird über Magnesiumsulfat getrocknet, filtriert und im Vakuum eingeengt. Man erhält 2-Benzylsulfanylmethyl-6-chlor-8,8a-dihydro-indeno[1,2-d]thiazol-3a-ol mit einem Schmelzpunkt von 102-104°C.

## Patentansprüche

1. Verbindungen der Formel I, 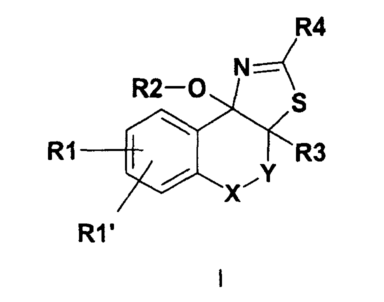 worin bedeuten
Y eine direkte Bindung;
X CH₂;
R1, R1' unabhängig voneinander H, F, Cl, Br, J, (C₁-C₆)-Alkyl;
R2 H, (C₁-C₆)-Alkyl;
R3 H, F;
R4 (C₈-C₁₆)-Cycloalkyl oder (CH₂)ₙ-A-R8, wobei n = 1 - 6 sein kann, wobei die Gruppe -(CH₂)₁₋₆-O-CH₂-Phenyl, worin Phenyl unsubstituiert ist, ausgenommen ist;
A O, S;
R8 (C₅-C₂₄)-Alkyl, oder (CH₂)ₘ-Aryl, wobei m = 0-6 und Aryl gleich Phenyl ist
und der Arylteil bis zu zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, SO₂-NH₂, SO₂-NH(C₁-C₈-Alkyl), SO₂-N(C₁-C₈-Alkyl)₂, SO₂-NH(C₃-C₈-Cycloalkyl), SO₂-N(C₃-C₈-Cycloalkyl)₂, (CH₂)m-SO₂-NH₂, (CH₂)ₘ-SO₂-NH-(C₁-C₆)-Alkyl, (CH₂)m-SO₂-N((C₁-C₆)-Alkyl)₂, wobei m = 1-6 sein kann, SO₂-N(=CH-N(CH₃)₂), (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH(C₁-C₆)-Alkyl, N(C₁-C₆-Alkyl)₂, NH-CO-(C₁-C₆)-Alky, NH-CO-Phenyl, NH-SO₂-(C₁-C₈-Alkyl), N(C₁-C₆-Alkyl)-SO₂-(C₁-C₈-Alkyl), NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl oder CONH₂ substituiert sein kann, Pyrrolidin-1-yl, Morpholin-1-yl, Piperidin-1-yl, Piperazin-1-yl, 4-Methylpiperazin-1-yl, (CH₂)p-Phenyl, O-(CH₂)ₚ-Phenyl, S-(CH₂)ₚ-Phenyl oder SO₂-(CH₂)ₚ-Phenyl, wobei p = 0-3 sein kann;
sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen gemäß Anspruch 1 und ein oder mehrere anorektische Wirkstoffe.

4. Verbindungen gemäß Anspruch 1 zur Anwendung als Medikament zur Prophylaxe oder Behandlung der Obesitas.

5. Verbindungen gemäß Anspruch 1 zur Anwendung als Medikament zur Prophylaxe oder Behandlung des Typ II Diabetes.

6. Verbindungen gemäß Anspruch 1 in Kombination mit mindestens einem weiteren anorektischen Wirkstoff zur Anwendung als Medikament zur Prophylaxe oder Behandlung der Obesitas.

7. Verbindungen gemäß Anspruch 1 in Kombination mit mindestens einem weiteren anorektischen Wirkstoff zur Anwendung als Medikament zur Prophylaxe oder Behandlung der des Typ II Diabetes.

8. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen gemäß Anspruch 1 zur Herstellung eines Medikaments zur Prophylaxe oder Behandlung der Obesitas.

10. Verwendung der Verbindungen gemäß Anspruch 1 zur Herstellung eines Medikaments zur Prophylaxe oder Behandlung des Typ II Diabetes.

11. Verfahren zur Herstellung der Verbindungen gemäß Anspruch 1, **dadurch gekennzeichnet, daß** man nach folgendem Formelschema 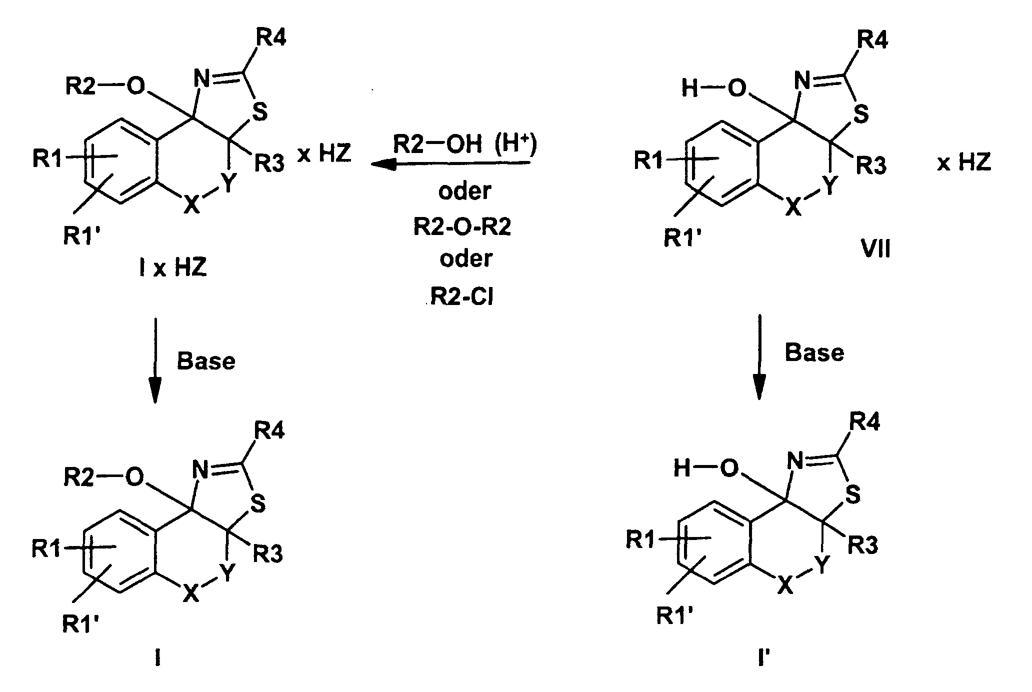 eine Verbindung der Formel VII, worin X, Y, R1, R1', R3, und R4 die in Anspruch 1 angegebenen Bedeutungen haben, entweder mit einer Base zur Verbindung I' umsetzt, worin R2 gleich Wasserstoff ist, oder mit einer der Verbindungen R2-OH, R2-O-R2 oder R2-CI, worin R2 die zu Formel I angegebene Bedeutung hat, zu einer Verbindung der Formel I x HZ umsetzt, die dann mit einer Base zur Verbindung der Formel I weiter umsetzt wird.

## Claims

1. A compound of the formula I, 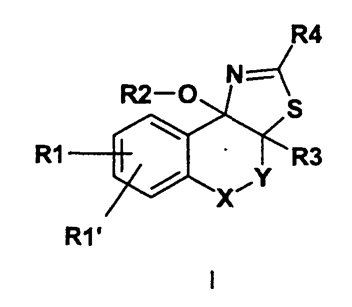 in which
Y is a direct bond;
X is CH₂;
R1, R1' independently of one another are H, F, Cl, Br, I, (C₁-C₆)-alkyl;
R2 is H, (C₁-C₆)-alkyl;
R3 is H, F;
R4 is (C₈-C₁₆)-cycloalkyl or (CH₂)ₙ-A-R8, where n can be 1-6, except for the group -(CH₂)₁₋₆-O-CH₂-phenyl in which phenyl is unsubstituted;
A is O, S;
R8 is (C₅-C₂₄)-alkyl, or (CH₂)ₘ-aryl, where m is 0-6 and aryl is phenyl
and the aryl moiety can be substituted up to two times by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, SO₂-NH₂, SO₂-NH(C₁-C₈-alkyl), SO₂-N(C₁-C₈-alkyl)₂, SO₂-NH(C₃-C₈-cycloalkyl), SO₂-N(C₃-C₈-cycloalkyl)₂, (CH₂)ₘ-SO₂-NH₂, (CH₂)ₘ-SO₂-NH-(C₁-C₆)-alkyl, (CH₂)ₘ-SO₂-N((C₁-C₆)-alkyl)₂, where m can be 1-6, SO₂-N(=CH-N(CH₃)₂), (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]₂, CONH(C₃-C₆)cycloalkyl, NH₂, NH(C₁-C₆)-alkyl, N(C₁-C₆-alkyl)₂, NH-CO-(C₁-C₆)-alkyl, NH-CO-phenyl, NH-SO₂-(C₁-C₈-alkyl), N(C₁-C₆-alkyl)-SO₂-(C₁-C₈-alkyl), NH-SO₂-phenyl, where the phenyl ring can be substituted up to two times by F, Cl, CN, OH, (C₁-C₆)-alkyl, O-(C₁-C₆)-alkyl, CF₃, COOH, COO(C₁-C₆)-alkyl or CONH₂;
pyrrolidin-1-yl, morpholin-1-yl, piperidin-1-yl, piperazin-1-yl, 4-methylpiperazin-1-yl, (CH₂)ₚ-phenyl, O-(CH₂)p-phenyl, S-(CH2)p-phenyl or SO₂-(CH₂)ₚ-phenyl, where p can be 0-3;
and its physiologically acceptable salts.

2. A pharmaceutical comprising one or more of the compounds as claimed in claim 1.

3. A pharmaceutical comprising one or more of the compounds as claimed in claim 1 and one or more anorectic active preparations.

4. A compound as claimed in claim 1 for use as a medicament for the prophylaxis or treatment of obesity.

5. A compound as claimed in claim 1 for use as a medicament for the prophylaxis or treatment of type II diabetes.

6. A compound as claimed in claim 1 in combination with at least one further anorectic active compound for use as a medicament for the prophylaxis or treatment of obesity.

7. A compound as claimed in claim 1 in combination with at least one further anorectic active compound for use as a medicament for the prophylaxis or treatment of type II diabetes.

8. A process for preparing a pharmaceutical comprising one or more compounds as claimed in claim 1, which comprises mixing the active compound with a pharmaceutically suitable vehicle and bringing this mixture into a form suitable for administration.

9. The use of the compounds as claimed in claim 1 for preparing a medicament for the prophylaxis or treatment of obesity.

10. The use of the compounds as claimed in claim 1 for preparing a medicament for the prophylaxis or treatment of type II diabetes.

11. A process for preparing the compounds as claimed in claim 1, which comprises reacting, according to the formula scheme below, 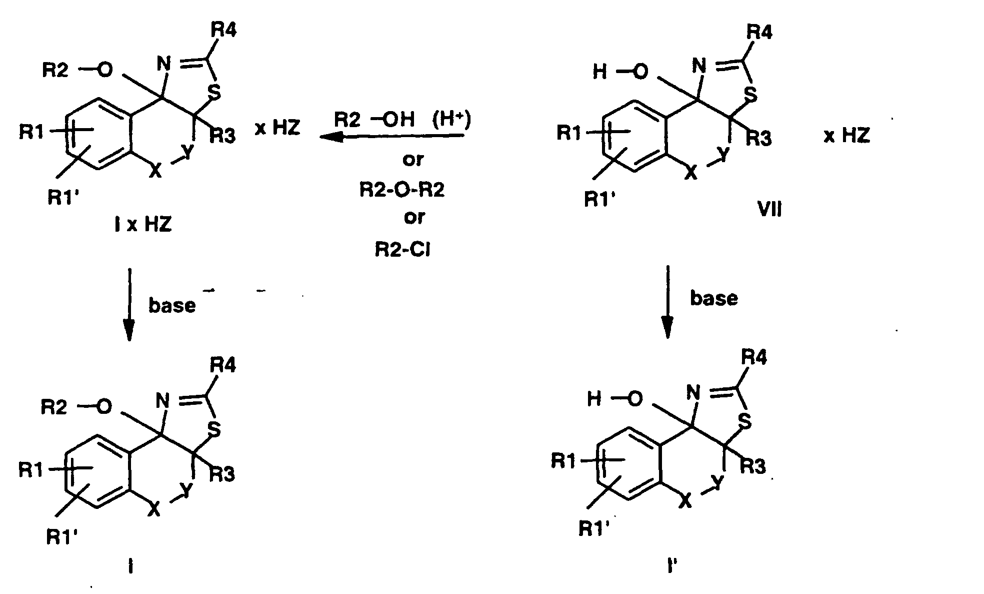 a compound of the formula VII in which X, Y, R1, R1', R3 and R4 are as defined in claim 1 either with a base to give compound I' in which R2 is hydrogen or with one of the compounds R2-OH, R2-O-R2 or R2-Cl in which R2 is as defined under formula I to give a compound of the formula I x HZ, which is then reacted further with a base to give the compound of the formula I.

## Revendications

1. Composés de formule I, 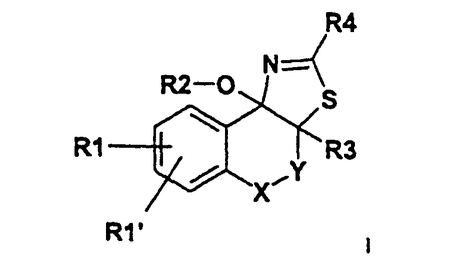 où
Y représente une liaison directe ;
X représente un groupe CH₂ ;
R1, R1' représentent indépendamment l'un de l'autre, des atomes de H, F, Cl, Br, I, un groupe alkyle en C₁-C₆ ;
R2 représente un atome de H, un groupe alkyle en C₁-C₆ ;
R3 représente un atome de H, F ;
R4 représente un groupe cycloalkyle en C₈-C₁₆ ou (CH₂)ₙ-A-R8, n peut être n = 1 à 6, le groupe -(CH₂)₁₋₆-O-CH₂-phényle, où le groupe phényle est non substitué est exclu ;
A représente un atome de O, S ;
R8 représente un groupe alkyle en C₅-C₂₄ ou (CH₂)ₘ-aryle, m = 0 à 6 et aryle est égal à un groupe phényle,
et le fragment aryle peut être substitué jusqu'à deux fois par des substituants F, Cl, Br, OH, CF₃, NO₂, CN, OCF₃, O-alkyle en C₁-C₆, S-alkyle en C₁-C₆, SO-alkyle en C₁-C₆, SO₂-alkyle en C₁-C₆, SO₂-NH₂, SO₂-NH-alkyle en C₁-C₈, SO₂-N(alkyle en C₁-C₈)₂, SO₂-NH-cycloalkyle en C₃-C₈, SO₂-N(cycloalkyle en C₃-C₈)₂, (CH₂)ₘ-SO₂-NH₂, (CH₂)ₘ-SO₂-NH-alkyle en C₁-C₆, (CH₂)ₘ-SO₂-N(alkyle en C₁-C₆)₂, m pouvant être m = 1 à 6, SO₂-N(=CH-N(CH₃)₂), alkyle en C₁-C₆, cycloalkyle en C₃-C₆, COOH, COO-alkyle en C₁-C₆, COO-cycloalkyle en C₃-C₆, CONH₂, CONH-alkyle en C₁-C₆, CON(alkyle en C₁-C₆)₂, CONH-cycloalkyle en C₃-C₆, NH₂, NH-alkyle en C₁-C₆, N(alkyle en C₁-C₆)₂, NH-CO-alkyle en C₁-C₆, NH-CO-phényle, NH-SO₂-alkyle en C₁-C₈, N(alkyl en C₁-C₆)-SO₂-(alkyle en C₁-C₈), NH-SO₂-phényle, le cycle phényle pouvant être substitué jusqu'à deux fois par des substituants F, Cl, CN, OH, alkyle en C₁-C₆, O-alkyle en C₁-C₆, CF₃, COOH, COO-alkyle en C₁-C₆ ou CONH₂, pyrrolidin-1-yle, morpholin-1-yle, pipéridin-1-yle, pipérazin-1-yle, 4-méthyl-pipérazin-1-yle, (CH₂)ₚ-phényle, O-(CH₂)ₚ-phényle, S-(CH₂)ₚ-phényle ou SO₂-(CH₂)ₚ-phényle, p pouvant être p = 0 à 3 ;
ainsi que leurs sels physiologiquement tolérés.

2. Médicament renfermant un ou plusieurs des composés selon la revendication 1.

3. Médicament renfermant un ou plusieurs des composés selon la revendication 1 et un ou plusieurs principes actifs anorectiques.

4. Composés selon la revendication 1 pour l'utilisation en tant que médicament pour la prévention ou le traitement de l'obésité.

5. Composés selon la revendication 1 pour l'utilisation en tant que médicament pour la prévention ou le traitement du diabète type II.

6. Composés selon la revendication 1 en combinaison avec au moins un autre principe actif anorectique pour l'utilisation comme médicament pour la prévention ou le traitement de l'obésité.

7. Composés selon la revendication 1 en combinaison avec au moins un autre principe actif anorectique pour l'utilisation comme médicament pour la prévention ou le traitement du diabète type II.

8. Procédé pour la préparation d'un médicament renfermant un ou plusieurs des composés selon la revendication 1, **caractérisé en ce qu'**on mélange le principe actif avec un véhicule approprié pour la pharmacie et ce mélange est mis en forme appropriée pour l'administration.

9. Utilisation des composés selon la revendication 1 pour la préparation d'un médicament pour la prévention ou le traitement de l'obésité.

10. Utilisation des composés selon la revendication 1 pour la préparation d'un médicament pour la prévention ou le traitement du diabète type II.

11. Procédé pour la préparation des composés selon la revendication 1, **caractérisé en ce qu'**on fait réagir selon le schéma de formules suivant 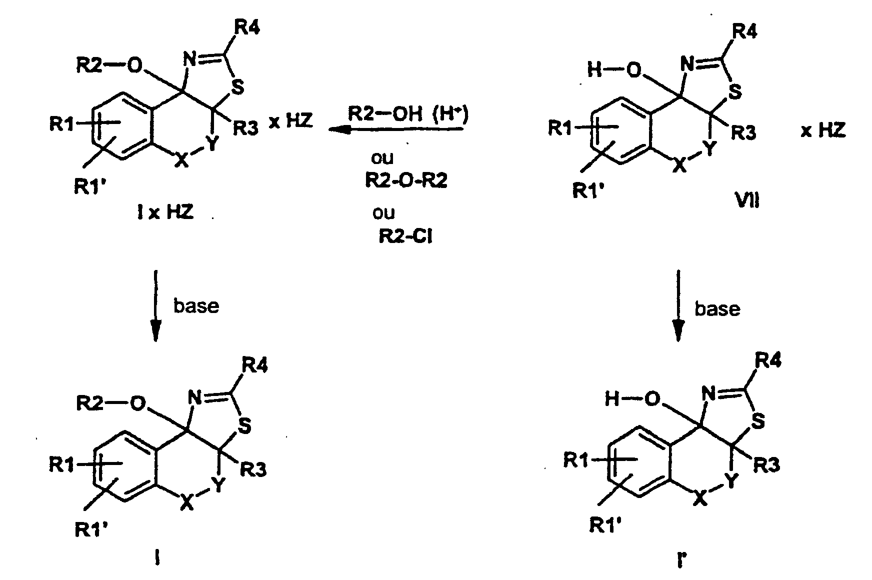 un composé de formule VII, où X, Y, R1, R1', R3 et R4 possèdent les significations données à la revendication 1, soit sur une base pour obtenir le composé de formule I', où R2 représente un atome d'hydrogène, soit sur un des composés R2-OH, R2-O-R2 ou R2-CI, où R2 possède la signification donnée à la formule I, pour donner un composé de formule I x HZ, que l'on fait alors réagir sur une base pour donner un composé de formule I.
